(19) Europäisches Patentamt
European Patent Office
Office européen des brevets

(11) **EP 3 112 396 A1**

(12) **EUROPEAN PATENT APPLICATION**
published in accordance with Art. 153(4) EPC

(43) Date of publication:
**04.01.2017 Bulletin 2017/01**

(51) Int Cl.:
**C08H 1/00** (2006.01)          **C08J 3/24** (2006.01)
**A61L 27/22** (2006.01)

(21) Application number: **14884248.7**

(22) Date of filing: **14.04.2014**

(86) International application number:
**PCT/CN2014/075298**

(87) International publication number:
**WO 2015/127711 (03.09.2015 Gazette 2015/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **28.02.2014   CN 201410071402**

(71) Applicant: **Union Hospital, Tongji Medical College,
Huazhong University of Science and Technology
Wuhan Hubei 430022 (CN)**

(72) Inventors:
• **WANG, Lin**
  **Wuhan**
  **Hubei 430022 (CN)**
• **ZHANG, Yeshun**
  **Wuhan**
  **Hubei 430022 (CN)**
• **HUANG, Lei**
  **Wuhan**
  **Hubei 430022 (CN)**

• **LIU, Jia**
  **Wuhan**
  **Hubei 430022 (CN)**
• **WANG, Zheng**
  **Wuhan**
  **Hubei 430022 (CN)**
• **LI, Yongkui**
  **Wuhan**
  **Hubei 430022 (CN)**
• **YANG, Wen**
  **Wuhan**
  **Hubei 430022 (CN)**
• **QI, Chao**
  **Wuhan**
  **Hubei 430022 (CN)**

(74) Representative: **Herrmann, Uwe
Lorenz Seidler Gossel
Rechtsanwälte Patentanwälte
Partnerschaft mbB
Widenmayerstraße 23
80538 München (DE)**

(54) **PREPARATION METHOD AND USE OF SERICIN HYDROGEL**

(57)    A method for preparing a sericin hydrogel, the method including: 1) weighing a cocoon of a fibroin-deficient mutant silkworm, *Bombyx mori,* extracting the cocoon by an aqueous solution of LiBr or LiCl, dialyzing an extracted solution to yield a sericin solution having a concentration of a non-degraded sericin of between 0.1 and 4 wt. %; and 2) concentrating the sericin solution to a concentration of between 1.5 and 10 wt. %, adding a crosslinking agent to the concentrated sericin solution at a ratio of between 2 and 500 μL of the crosslinking agent per each milliliter of the sericin solution, fully blending the crosslinking agent with the concentrated sericin solution, and keeping a resulting mixture at the temperature of between 4 and 45°C for between 5 s and 36 hrs to yield a hydrogel.

**EP 3 112 396 A1**

**Description**

FIELD OF THE INVENTION

The invention relates to the field of a biomedical composite material, and more particularly to methods of preparing and using sericin hydrogel. The hydrogel, featuring biological activity and versatility, can be employed as a carrier between growth factors, medicines and cells, and be applied in the treatments and recovery from multiple soft tissue injuries, comprising skin injury, muscle injury, vessel injury, nerve injury and myocardial injury, etc.

BACKGROUND OF THE INVENTION

Hydrogel is a three-dimensional network polymer which can swell in the water and retain a great amount of water. Hydrogel, as a crucial biomaterial, featuring swellability and water-retaining property, is widely applied in biomedical and tissue engineering fields. One of the hydrogel is produced by natural biomaterial (such as collagen, alginate, and chitosan, etc.), and the hydrogel gains popularity as the hydrogel features a highly similar physicochemical property with engine body, and excellent biological property. Recently, new hydrogel production using natural biomaterial has become an important subject and hot topic in tissue engineering field.

Silk sericin is a natural macromolecule adhesive protein which is sleeved on the surface of silk fiberoin, accounting for about 20-30% of cocoon. Silk sericin is composed of polypeptides with a molecular weight of 24-400 kDa, and the molecule of the polypeptides is composed of 18 types of amino acid such as serine, asparaginic acid, and glycine etc. For a long time, people have limited recognition and research on silk sericin, leading to a major waste of the precious natural resource in silk reeling industry. In recent years, people discovered that silk sericin features biological properties such as moisture retention, antibiosis, antioxidant, anticoagulation and promoting cell adhesion and proliferation, ect. In addition, silk sericin, featureing hydrophilia and biodegradability, is an ideal biomedical material. It is reported that silk sercin is often used to produce biological scaffold with high performance via copolymerization or simple crosslinking with other materials (such as elastin, and poval etc.) However, biomaterial made only from silk sericin is limited to fragile two-dimensional silk sericin film. A three-dimensional pure silk sericin hydrogel in a real sense is still hard to realize.The main reason is that traditional method to separate silk sericin (such as high temperature and alkaline process) can easily cause silk sericin degradation, and degraded silk sericin has a small molecular weight, fragile physical property and high water solubility, thus hydrogel is hard to produce( including crosslinking). Application of silk sericin in tissue engineering is limited. Therefore, research on a pure silk sericin hydrogel which features high biocompatilibility, excellent mechanical properties and stability is of great social significance and has a promising application. So whether the pure silk sericin hydrogel with a three-dimensional structure can be produced and be applied in biochemical field is an important subject worthy of studying.

The invention employs a cocoon which is a mutant variety lack of bombyx mori silk fibroin to extract pure silk sericin via lithium bromide extraction, and uses a method of chemical covalent crossilinking for the first time to produce an injectable pure silk sericin hydrogel which features fluorescence property, excellent biocompability, cell adhesivity , and excellent mechanical property. The fluorescence property of the silk sericin hydrogel can be used to real-time tracking and imaging inside a body.

The three-dimensional pure silk sericin hydrogel, facilitating biological activity and versatility, can be employed as a carrier between growth factors, medicines and cells, and be applied in the treatments and recovery from multiple soft tissue injuries, comprising skin injury, muscle injury, vessel injury, nerve injury and myocardial injury, etc.

SUMMARY OF THE INVENTION

In view of the above-described problems, it is one objective of the invention to provide methods of preparing and using sericin hydrogel. The fibroin-deficient mutant silkworm, *Bombyx mori,* is utilized as the raw material. A sericin solution is prepared by extraction and purification of the raw material and then crosslinked by a crosslinking agent (aldehydes and geniposide) so as to obtain the sericin hydrogel. The sericin hydrogel possesses cell compatibility, cell adhesion, high crosslinking rate, quick gelation, good mechanical performance, stable properties, and intrinsic fluorescence. The sericin hydrogel is a new type biomaterial, which can be utilized as a growth factor, a drug, and a cell carrier, and is applicable for repairing many kinds of soft tissue injuries and treating diseases, including but not limited to skin injury, muscle injury, vascular injury, nerve injury, and myocardial injury.

To achieve the above objective, in accordance with one embodiment of the invention, there is provided a method for preparing a sericin hydrogel, the method comprising:

1) weighing a cocoon of a fibroin-deficient mutant silkworm, *Bombyx mori,* extracting the cocoon by an aqueous solution of LiBr or LiCl, dialyzing an extracted solution whereby yielding a sericin solution having a concentration of a non-degraded sericin of between 0.1 and 4 wt. %; and

2) concentrating the sericin solution to the concentration of between 1.5 and 10 wt. %, adding a crosslinking agent to the concentrated sericin solution at a ratio of between 2 and 500 μL of the crosslinking agent per each milliliter of the sericin solution, fully blending the crosslinking agent with the concentrated sericin solution, and keeping a resulting mixture at a temperature of between 4 and 45°C for between 5 s and 36 hrs whereby yielding a hydrogel.

In a class of this embodiment, the crosslinking agent is selected from the group consisting of glutaraldehyde, malondialdehyde, and geniposide.

In a class of this embodiment, a concentration of the crosslinking agent is between 1 and 25 wt. %.

In a class of this embodiment, in 1), the sericin solution is prepared as follows:

a) weighing the cocoon of the fibroin-deficient mutant silkworm, *Bombyx mori,* cutting the cocoon into pieces, washing and dehydrating the cocoon pieces;

b) immersing the cocoon pieces obtained from step a) into the aqueous solution of LiBr or LiCl at the temperature of between 25 and 50°C for dissolving the sericin, wherein each gram of the cocoon pieces corresponds to between 20 and 100 mL of the aqueous solution of LiBr or LiCl having a concentration of between 6 and 8 mol/L;

c) centrifuging a mixture of step b), removing insoluble substances therefrom whereby yielding a clarified solution;

d) adding a Tris-HCl buffer solution having a concentration of 1 mol/L and a pH value of between 8.0 and 11.0 to the clarified solution obtained in c) with a volume ratio of the Tris-HCl buffer solution to the clarified solution of 1:4, dialyzing a mixed solution against a hyperpure water whereby yielding the sericin solution; and

e) removing a precipitate from the sericin solution by centrifuging, and concentrating the sericin solution to the concentration of between 1.5 and 10 wt. %.

In a class of this embodiment, the sericin hydrogel is prepared as follows:

a) weighing the cocoon of the fibroin-deficient mutant silkworm, cutting the cocoon into pieces in a size of 1 cm$^2$, washing the cocoon pieces three times, and dehydrating the cocoon pieces;

b) immersing the cocoon pieces obtained from step a) into the aqueous solution of LiBr at a temperature of 35°C for 24 hrs for dissolving the sericin, wherein each gram of the cocoon pieces corresponds to 40 mL of the aqueous solution of LiBr having a concentration of 6 mol/L;

c) centrifuging a mixture of step b), removing insoluble substances therefrom whereby yielding a clarified solution;

d) adding a Tris-HCl buffer solution having a concentration of 1 mol/L and a pH value of 9.0 to the clarified solution obtained in c) with a volume ratio of the Tris-HCl buffer solution to the clarified solution of 1:4, dialyzing a mixed solution whereby yielding the sericin solution;

e) removing a precipitate from the sericin solution obtained in d) by centrifuging, and concentrating the sericin solution to the concentration of between 1.5 and 10 wt. %; and

f) adding glutaraldehyde to the sericin solution, blending glutaraldehyde with the sericin solution, and keeping a resulting mixture at 37°C for 5 min whereby yielding a hydrogel, wherein each milliliter of the sericin solution corresponds to between 2 and 100 μL of between 20 and 25 wt. % of glutaraldehyde.

In accordance with another embodiment of the invention, there is provided a method for using the sericin hydrogel in biomedical materials.

In a class of this embodiment, the sericin hydrogel is used in the following aspects:

1) damage repair and disease treatment including, but not limited to, skin injury, muscle injury, vascular injury, nerve injury, and myocardial injury;

2) tissue repair by combining the sericin hydrogel with tool cells to pack corresponding treating factors; and

3) as a growth factor, a drug, a cell carrier, or a scaffold.

In accordance with still another embodiment of the invention, there is provided a method for using the sericin hydrogel in fluorescent probe materials.

In accordance with another embodiment of the invention, there is provided a method for preparing a lyophilized sericin scaffold, the method comprising:

1) freezing the sericin hydrogel at a temperature beneath zero; and

2) vacuum drying the frozen sericin hydrogel whereby yielding a lyophilized sericin scaffold.

In accordance with still another embodiment of the invention, there is provided a method for using the lyophilized sericin scaffold in biochemical materials.

Advantages according to embodiments of the invention are summarized as follows:

1) The conventional sericin is originated from the wild type silkworm cocoon and is treated with the conventional extracting means, which however results in serious degradation of the sericin and poor gelation property (when the high temperature high pressure method or the alkali method is adopted) or results in difficulties in separation of the sericin from the fibroin (when the extraction by lithium bromide solution is adopted). In contrast, the method of the invention is able to keep the excellent natural property of the sericin and therefore overcomes the difficulties in obtaining the non-degraded sericin with high biological character.

2) The extraction efficiency of the sericin by the method exceeds 90%.

3) The method adopts the non-degraded sericin solution and the aldehydes or the geniposide to prepare the hydrogel for the first time. The preparation of the hydrogel is simple and feasible.

4) No injectable pure 3D sericin hydrogel has been developed before the invention, and composite biological materials, such as gelatin, polyvinyl alcohol, and chitosan are generally prepared by simply blending the sericin with other materials. The method of the invention successfully prepared the 3D scaffold of the pure sericin with excellent mechanical performance and the injectable pure sericin hydrogel for the first time.

5) The sericin hydrogel prepared by the method of the invention possess unique property. The complete sericin peptide extracted by the method keeps the excellent nature property of the sericin, and the sericin solution is easily crosslinked to form the gel under the action of the crosslinking agent (the aldehydes or the geniposide). The sericin hydrogel prepared by the method of the invention possesses features of the conventional hydrogel, such as the porosity and the degradability, as well as the following unique features: I) excellent biocompatibility for carrying multiple type of cells and support the cell adhesion and proliferation. II) injectability and in situ gelation, so that the material can be transferred *in vivo* by injection and the injury caused by injection is much smaller than surgery. III) intrinsic fluorescence enables the hydrogel to be traced *in vivo* in real time; IV) good mechanical performance (the sericin hydrogel possesses much better mechanical performance than the alginate hydrogel for tissue engineering); V) the degraded product has strong buffer capacity for neutralizing the pH value, so that when the material is used as the carrier for a drug or the growth factor, the drug or the growth factor is prevented from deactivating under the influence of the pH environment; VI) the degradation rate is responsive to the pH value; and VII) excellent drug delivery ability and excellent drug carrier.

6) Both the crosslinking rate and the gelation time of the sericin hydrogel obtained by the method of the invention are controllable (the in situ gelation can be realized according to requirements), and the crosslinking rate and the gelation rate are controlled by changing the dose and the type of the crosslinking agent or the concentration of the sericin solution.

7) The sericin hydrogel obtained by the method of the invention has wide use and can be prepared into gels of different shapes and into porous bioscaffold with different shapes and pore diameter by lyophilization.

8) The sericin hydrogel possesses excellent biocompatibility and cell adhesion and supports survival and proliferation of many types of cells. The sericin hydrogel has excellent controlled drug release. The sericin hydrogel and the 3D porous bioscaffold of the sericin can be used as extracellular matrix to support the cell growth and promote the nutrients exchange. Relevant experiment results indicate that the sericin hydrogel is applicable to but not limited to the damage repairs and disease treatment in blood vessels, skin, muscle, skin, nerves, and the like.

BRIEF DESCRIPTION OF THE DRAWINGS

The invention is described hereinbelow with reference to the accompanying drawings, in which:

FIG. **1** illustrates sericin nerve conduits and lyophilized scaffold acquired by lyophilization and vacuum drying of sericin hydrogel at different temperatures;

FIG. **2** is a chart showing relation between a concentration of a sericin protein and a gelation time (at a room temperature);

FIG. **3** is a chart showing a crosslinking degree of a sericin hydrogel at a temperature of 37°C;

FIGs. **4A-4B** are block diagrams representing an elasticity modulus of a sericin hydrogel and a alginate hydrogel, in which, FIG. **4A** represents a compressive strength, and FIG. **4B** represents the elasticity modulus;

FIG. **5** is a curve chart showing a swelling rate of a sericin hydrogel at different pH environment at a temperature of 37°C;

FIG. **6** is a curve chart showing degradation kinetics of a sericin hydrogel in a PBS solution at a temperature of 37°C;

FIG. **7** illustrates effect of a degraded product of a sericin hydrogel on a pH value of a PBS solution;

FIG. **8** illustrates water saturation of a sericin hydrogel at different pH values (3.0, 7.4, and 11.0);

FIG. **9** is analysis spectra of a sericin by differential scanning calorimetry, in which (I) is a sericin protein, (II) is a sericin hydrogel crosslinked by glutaraldehyde, (III) is a sericin treated by ethanol, and (IV) is a sericin treated by glutaraldehyde and concentrated hydrocholoric acid;

FIG. **10** is infrared spectra of a sericin hydrogel and a sericin protein, in which, (a) is a is a sericin treated by ethanol, (b) is a a sericin treated by glutaraldehyde and concentrated hydrocholoric acid, (c) is a sericin hydrogel crosslinked by glutaraldehyde, and (d) is a sericin protein;

FIG. **11** shows pictures of sericin conduits and lyophilized hydrogel observed under a fluorescence microscope (A-H represent conduits, and I-L represent lyophilized scaffolds of sericin hydrogels);

FIG. **12** shows mice injected with sericin hydrogel for tracing by a small animal imaging system;

FIG. **13** is a curve chart showing release of horseradish peroxidase (HRP) from a sericin hydrogel;

FIGs. **14A-B** are cell adhesion and cell viability of human umbilical vein endothelial cells (HUVECs) in a sericin hydrogel group and a control group, in which, FIG. **14A** illustrates cell adhesion of the HUVECs on a sericin hydrogel (cell strain ECV304 is adopted), and FIG. **14B** illustrates cell viability of HUVECs (cell strain EA.hy926 is adopted);

FIGs. **15A-B** are adhesion and proliferation of the HUVECs in a sericin hydrogel group and a control group, in which, FIG. **15A** shows ordinary electron microscope pictures, and FIG. **15B** shows confocal laser scanning microscope pictures;

FIG. **16** illustrates adhesion and proliferation conditions of human skin epidermal cells (HaCaT) in a sericin hydrogel group and a control group;

FIG. **17** illustrates adhesion and proliferation conditions of mouse myoblasts (C2C12) in a sericin hydrogel group and a control group;

FIG. **18** illustrates adhesion and proliferation conditions of human embryo kidney cells (HEK293) in a sericin hydrogel group and a control group;

FIG. **19** illustrates adhesion and proliferation conditions of human primary embryo skin fibroblasts (CCC-ESF-1) in a sericin hydrogel group and a control group;

FIG. **20** illustrates adhesion and proliferation conditions of mouse microglia cells (BV2) in a sericin hydrogel group and a control group;

FIG. **21** illustrates adhesion and proliferation conditions of mouse islet endothelial cells (MS1) in a sericin hydrogel group and a control group;

FIG. **22** illustrates adhesion and proliferation conditions of rat Schwann cells (RSC926) in a sericin hydrogel group and a control group; and

FIG. **23** illustrates adhesion and proliferation conditions of rat cardiac myocytes (H9C2) in a sericin hydrogel group and a control group.

DETAILED DESCRIPTION OF THE EMBODIMENTS

For further illustrating the invention, experiments detailing methods of preparing and using sericin hydrogel are described below. It should be noted that the following examples are intended to describe and not to limit the invention.

**Example 1**

A method for preparing a sericin hydrogel was performed as follows:

1. Selection of silkworm cocoon

Cocoons of a fibroin-deficient mutant silkworm, *Bombyx mori,* (purchased from Sericultural Research Institute, Chinese Academy of Agricultural Sciences and conserved in the National Silkworm Resources Conservation Center therein) was adopted a raw material, and the silkworm cocoon mainly contains sericin.

2. Extraction and isolation of sericin protein

1) 1 g of the fibroin-deficient mutant silkworm cocoon (purchased from the Sericultural Research Institute, Chinese Academy of Agricultural Sciences) was cut into pieces with a size of 1 cm$^2$ and then placed in a clean Bunsen beaker. The pieces were washed by hyperpure water three times and centrifuged at a rotational speed of 3500 rpm for 5 min to remove the water therefrom.

2) 30-60 mL of an aqueous solution of LiBr having a concentration of 6 mol/L was added to the cocoon pieces obtained in 1). Thereafter, the beaker was placed in a water bath at a constant temperature of 35°C for 24 hrs for dissolving the sericin.

3) A resulting solution acquired in 2) was transferred to a centrifuge tube and centrifuged at the rotational speed of 35°C for 5 min to remove insoluble substances, and a clarified solution was yielded.

4) A Tris-HCl buffer solution having a concentration of 1 mol/L and a pH value of 9.0 was added to the clarified solution obtained in 3) with a volume ratio of the Tris-HCl buffer solution to the clarified solution of 1:4.

5) A resulting solution acquired in 4) was transferred to a pretreated dialysis bag (MWCO 3500). After two ends of the dialysis bay were then clamped by clips, the dialysis bay was placed in a beaker containing hyperpure water. The beaker was then placed on a stirrer, where the beaker was stirred for dialysis for 48 hrs and water was refreshed every 3 hrs.

6) The sericin solution obtained from the dialysis of step 5) was then transferred to a centrifuge tube and centrifuged at the rotational speed of 4000 rpm for 5 min, and a precipitate was then removed.

7) The sericin solution was added to the dialysis bag again and the two ends of the dialysis bag were clamped. The dialysis bag was then concentrated in a 20 wt. % PEG6000 solution until the sericin solution was concentrated to a desired concentration (above 2.0 wt. %).

8) The measurement of the protein adopted the bovine serum albumin (BSA) method and the drying method of the sericin solution.

9) The measurement of the protein was carried out according to SDS-PAGE. 10-15 $\mu$L of the sericin solution was utilized for the molecular weight measurement, and the remaining sericin solution was kept at a 4°C refrigerator before use.

3. Preparation of sericin hydrogel

1) The concentrated sericin solution was diluted to a concentration of 2.0 wt. % with the hyperpure water.

2) 22 $\mu$L of 25 wt. % glutaraldehyde was added to every 1 mL of the sericin solution.

3) A resulting mixture was fully stirred at a temperature of 37°C for 5 min so that the hydrogel was formed.

4. Experimental analysis

1) Analysis of gelation time and crosslinking rate of sericin

The sericin hydrogel was prepared by mixing the sericin solution and 25 wt. % glutaraldehyde at the room temperature according to a volume ratio of 100:20, the gelation time and the crosslinking degree were examined and recorded.

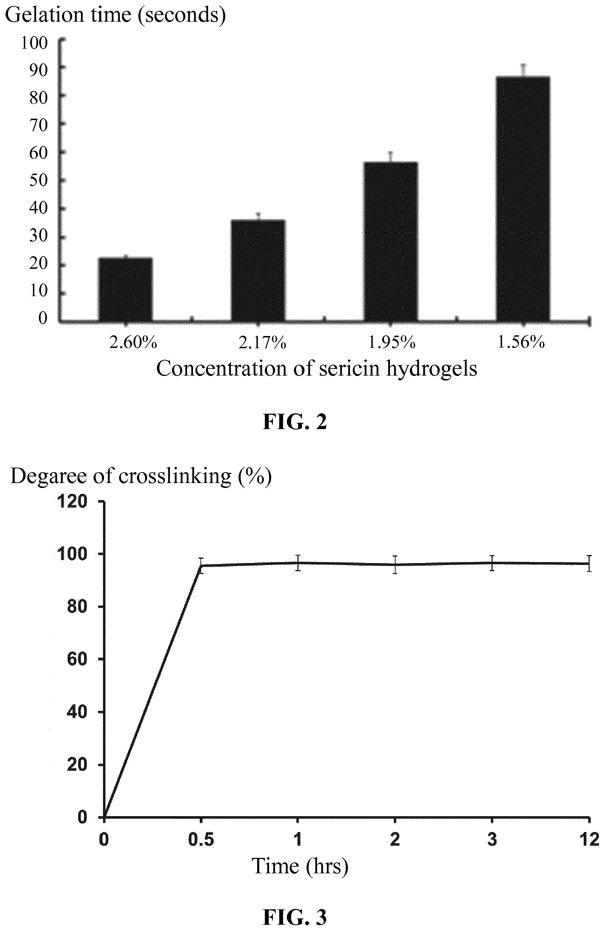

As shown in FIG. 2, the time for the crosslinking was prolonged with the decrease of the concentration of the sericin, which indicated that the gelation time could be regulated by the concentration of the sericin protein.

As shown in FIG. 3, the sericin solution was crosslinked by glutaraldehyde, the crosslinking was basically accomplished in 0.5 hr, and thereafter the crosslinking rate kept in a relatively constant state.

2) Porous structure of sericin hydrogel

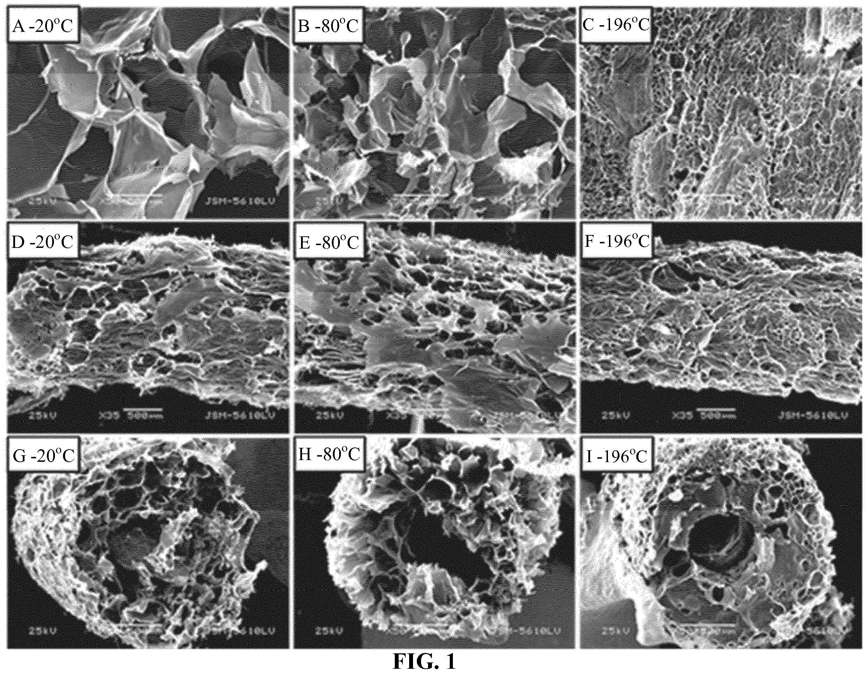

The sericin hydrogels were lyophilized at temperatures of -20°C, -80°C, and -196°C and examined under a scanning electron microscope (SEM). As shown in FIG. 1, pore diameters the 3D bioscaffold of the sericin acquired by lyophilizing the sericin hydrogels at -196°C, -80°C, and -20°C were 20 $\mu$m, 300 $\mu$m, and 700 $\mu$m, respectively. Thus, when the lyophilized temperature decreased, the pore diameter of the 3D bioscaffold was correspondingly reduced. Moreover, 3D bioscaffold contained a plurality of micropores which functioned as extracellular matrix for supporting the cell growth and promoting the nutrients exchange.

3) Comparison of mechanical performance between sericin hydrogel and alginate hydrogel

A cylindrical hydrogel sample with certain specifications was prepared, and a miniature universal test machine (Instron5848 MicroTester, Instron, USA) was used to measure the mechanical performance of the sericin hydrogel at the room temperature.

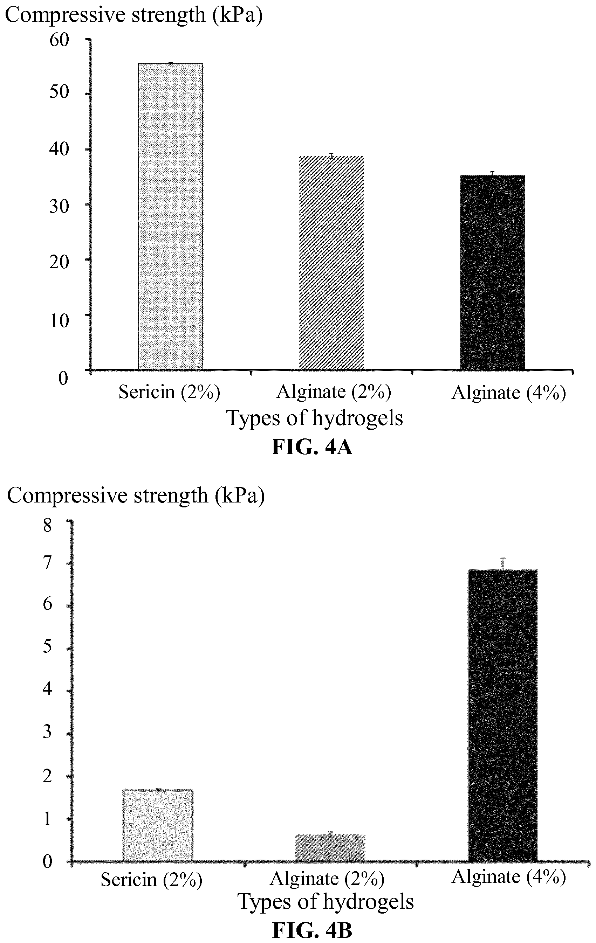

As shown in FIGs. 4A-4B, compared with the alginate hydrogel samples (having concentrations of 2 wt. % and 4 wt. % and containing equal weights of the alginate with large molecular weight and small molecular weight, respectively) which was widely used as the material in the tissue engineering research, the sericin hydrogel (having the concentration of 2 wt. %) has much excellent mechanical performance. The alginate hydrogel was prepared by adding 40 $\mu$L of a suspension of Ca$_2$SO$_4$ (0.21 g/mL) to 1 mL of an alginate solution and stirring.

4) Degradation rate of sericin hydrogel in different pH environments

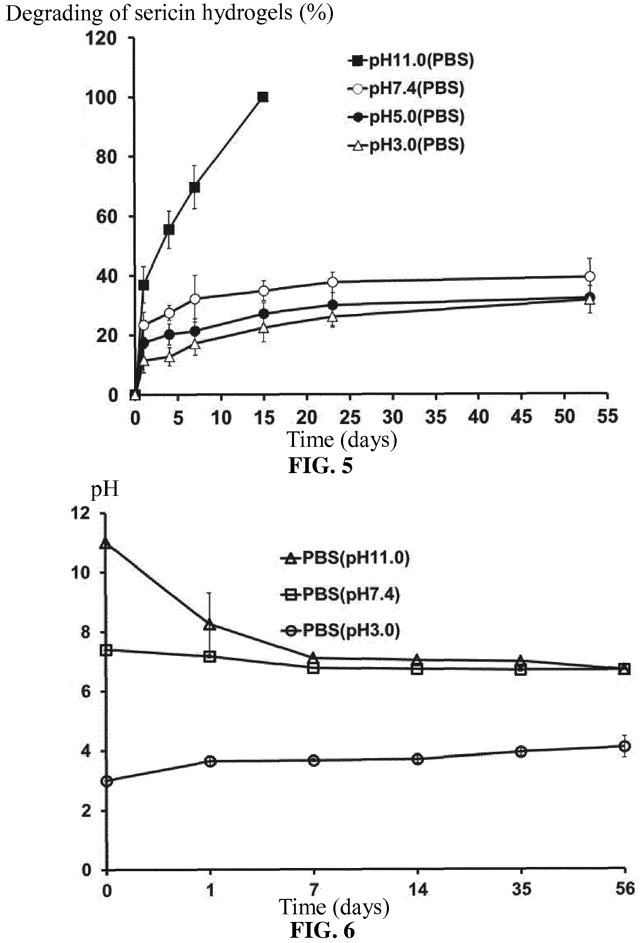

To know the influence of the pH environment on the degradation of the sericin hydrogel, the sericin hydrogels were immersed into phosphate buffer saline (PBS) solutions with different pH values (pH 3.0, pH 5.0, pH 7.4, and pH 11.0), respectively. The PBS solutions were refreshed every day, and the sericin hydrogels were taken out at different time points, dried, and weighed, and results were illustrated in FIG. **5**.

As shown in FIG. **5**, the degradation rate of the sericin hydrogel was relatively high in the former five days and then tended to be stable five days later. The degradation of the hydrogel is responsive to the pH value, in which, the sericin hydrogel had the highest degradation rate in the alkaline condition at the pH value of 11.0 and was totally degraded in 15 days; whereas the hydrogel had the lowest degradation rate at the acidic condition at the pH value of 3.0 and the degradation rate was only 30 wt. % after 53 days.

To test the influence of the degraded product of the sericin hydrogel on the pH value of the environment, the PBS solution was not refreshed and the pH value of the buffer solution was measured at corresponding time points, results of which were illustrated in FIG. **6**.

As shown in FIG. **6**, the degraded product of the sericin hydrogel was able to neutralize the pH value of the buffer solution.

5) Influence of pH value on swelling rate of the sericin hydrogel

The sericin hydrogels were lyophilized, weighed, and immersed to three PBS solutions with different pH values (pH 3.0, pH 7.4, and pH 11.0), and the swelling rates of the sericin hydrogels were measured at different time points according to the following equation:

$$swelling(\%) = \frac{Ws - Wd}{Wd} \times 100$$

in which, *Ws* represents a weight of the hydrogel in a swelling state, and *Wd* represents a dry weight thereof.

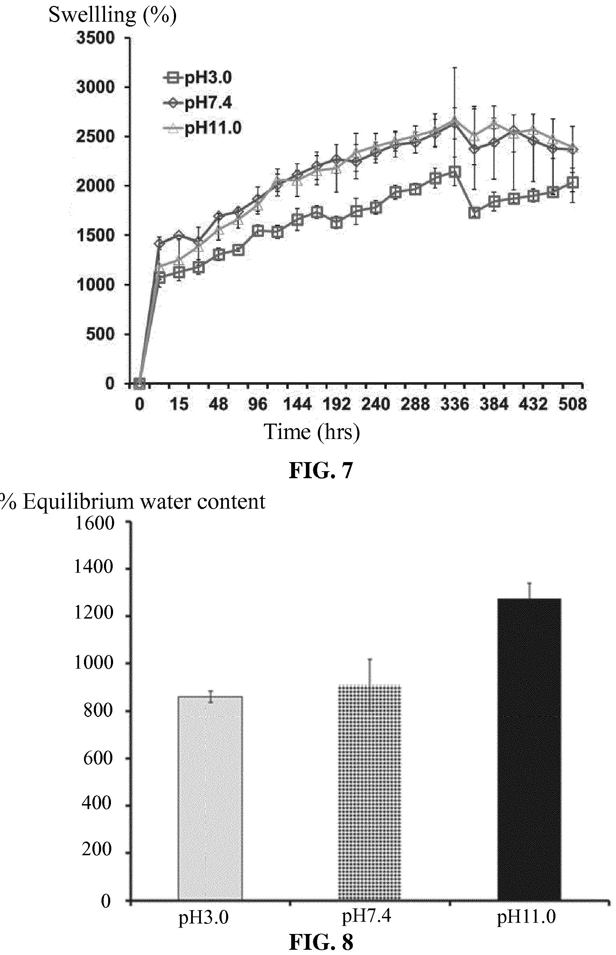

As shown in FIG. **7**, the swelling rate of the sericin hydrogel was largely increased in the former 3 hrs and tended to be stable two weeks later. A maximum swelling rate of the hydrogel reached 26 times at the pH values of 7.4 and 11.0; while a maximum swelling rate was 21 times at the pH value of 3.0.

6) Water saturation of sericin hydrogel at different pH values

The sericin hydrogel was frozen at a temperature of -80°C for overnight and dried in a vacuum drier, the dried samples were weighed and immersed into deionized water of different pH values (pH 3.0, pH 7.4, and pH 11.0) for 48 hrs. Swelling samples after water absorption were immediately weighed after removing the surface water, and the water saturation of each sample was obtained by calculating a ratio of the weight of the swelling sample after the water absorption to the dry weight of the sample.

As shown in FIG. **8**, the water saturation of the sericin hydrogel at different pH values (pH 3.0, pH 7.4, and pH 11.0) was illustrated. The water absorption of the sericin was 12.75% in the alkaline condition at the pH value of 11.0; and the water absorption of the sericin was 860% in the acidic condition at the pH value of 3.0.

7) Differential scanning calorimetry analysis of sericin

Changes of the fusion point in phase transition of the sericin hydrogel were measured using the differential scanning calorimetry analysis (adopting apparatus NETZSCH STA 449 F3, Germany). A flow rate for the measurement was 60 mL/min, and a programmed range for temperature increase was 40 -550°C, and the temperature was increased by 10°C every minute.

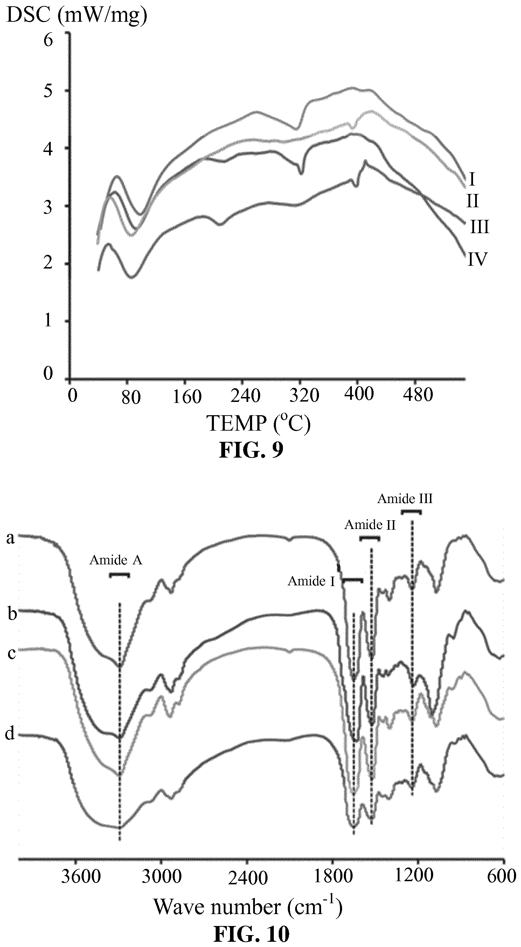

Thermal degradation chart of the sericin samples treated with different means was shown in FIG. **9**, in which, (I) represented lyophilization powder of pure sericin, (II) represented sericin treated by glutaraldehyde and concentrated hydrocholoric acid, (III) represented sericin treated by ethanol, and (IV) represented sericin hydrogel crosslinked by glutaraldehyde. Degradation temperatures of the sericin samples were as follows: 321.7°C of (I), 394.0°C of (II), 14.4°C of (IV), and 208.3°C and 398.3°C of (III).

8) Infrared spectrum analysis of sericin hydrogel and sericin

Fourier transform infrared spectroscopy (Nexus, Thermal Nicolet, USA) was utilized to measure a characteristic peak the sericin hydrogel at 4000-650 cm$^{-1}$.

As shown in FIG. **10**, in the sericin hydrogel crosslinked by glutaraldehyde, the secondary structure of the sericin had no obvious change, and the sericin hydrogel was able to keep the conformation of the natural sericin. Curve (a) represented the sericin treated by ethanol, curve (b) represented the sericin treated by glutaraldehyde and concentrated hydrocholoric acid, curve (c) represented the sericin hydrogel crosslinked by glutaraldehyde, and curve (d) represented lyophilization powder of the pure sericin.

It was known from the characteristic peaks amide I and amide II that the curve (c) and the curve (d) were basically the same, which meant that the secondary structure of the polypeptide in the sericin hydrogel was similar to that of the pure sericin, and therefore the sericin hydrogel was able to well maintain the natural confirmation of the sericin.

9) Detection of cell adhesion (ECV304) and cell viability (EA.hy926) of human umbilical vein endothelial cells (HUVECs) in the sericin hydrogel group and the control group

   a) The sericin solution was mixed with the crosslinking agent and spread in a plastic cell culture dish. After a stable gelation, the cell culture dish spread with the sericin hydrogel was washed by a sterilized PBS solution three times, immersed in 75% ethanol for 1 hr, and washed again by the sterilized PBS solution once. The treated culture dish was kept in the 4°C refrigerator before use.

b) Cells collected from a cell culture flask were suspended and blown and then seeded to the pretreated culture dish, and a culture dish not spread with the sericin hydrogel was utilized as a control group. The cells were cultured in high glucose DMEM media in a cell incubator (37°C, 5% $CO_2$, 100% humidity).

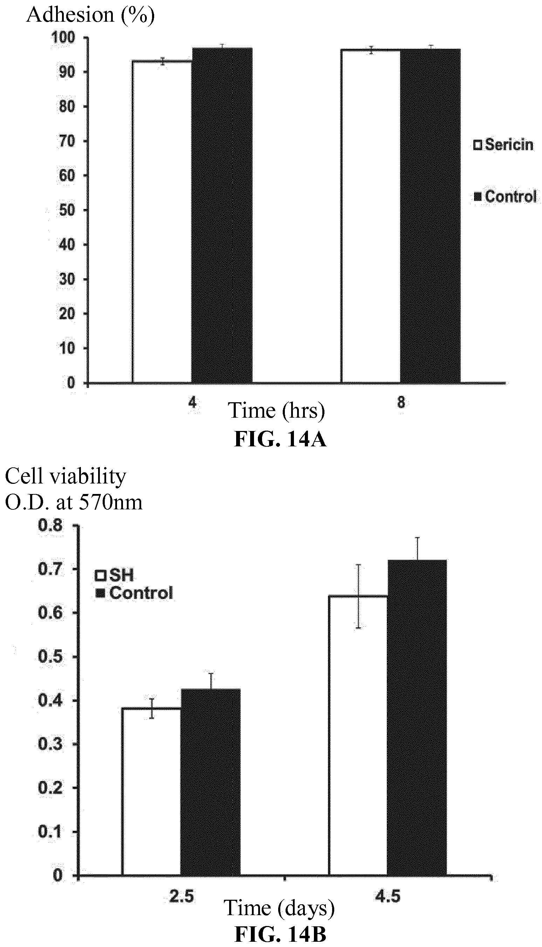

c) As shown in FIG. **14A**, non-adherent cells were counted at two time points, i. e., 4 hrs and 8 hrs after the seeding, and the cell adhesion rate was equal to a ratio of a difference between the original cell number and the non-adherent cell number to the original cell number. As shown in FIG. **14B**, cell viabilities were measured by MTT method after 2.5 days and 4.5 days of culture.

As shown in FIG. **14**, the sericin hydrogel had excellent cell adhesion to the HUVECs and was able to support survival and proliferation of cells, which demonstrated that the sericin hydrogel possessed excellent biocompatibility and cell adhesion.

**Example 2**

The method for preparing the sericin hydrogel is the same as that of Example 1 except that

1. 100 $\mu$L of 25 wt. % glutaraldehyde was added to every 1 mL of the sericin solution; and

2. A resulting mixture was fully mixed at the temperature of 37°C for 0.5 hr, so that the sericin hydrogel was obtained.

**Example 3**

The method for preparing the sericin hydrogel is the same as that of Example 1 except that

1. 2 $\mu$L of 25 wt. % glutaraldehyde was added to every 1 mL of the sericin solution; and

2. A resulting mixture was fully mixed at the temperature of 37°C for 0.5 hr, so that the sericin hydrogel was obtained.

**Example 4**

The method for preparing the sericin hydrogel is the same as that of Example 1 except that

1. 0.25 mL of 2 wt. % geniposide was added to every 1 mL of the sericin solution; and

2. A resulting mixture was fully mixed at the temperature of 37°C for 2 hrs, so that the sericin hydrogel was obtained.

**Example 5**

1. Preparation of lyophilized sericin scaffold

The hydrogels obtained from Example **1** were immediately frozen at temperatures of -20°C, -80°C, and -196°C, respectively, for 24 hrs, and then placed in the vacuum drier for drying (the drying time was determined according to the size of the sample), so that lyophilized sericin scaffolds with different pore diameters were yielded. The pore diameters were 316 nm, 167 nm, and 20.56 nm, respectively.

2. Experimental analysis

1) Microscope structures of the 3D sericin bioscaffolds at different temperatures

The sericin hydrogels were frozen at different temperatures (-20°C, -80°C, and -196°C), dried, and examined under SEM.
As shown in FIG. **1**, the pore diameters of the 3D porous bioscaffolds acquired by freezing the sericin hydrogels at -196°C, -80°C, and -20°C and lyophilizing the frozen sericin hydrogels were 20 $\mu$m, 300 $\mu$m, and 700 $\mu$m, respectively. Thus, the pore diameter of the 3D bioscaffold was reduced as the lyophilized temperature decreased. Besides, the 3D porous bioscaffold exists with quantities of micropores, thereby being adaptable to support the cell growth and promote the nutrient exchange as the extracellular matrix.

2) Detection of intrinsic fluorescence of sericin conduit and lyophilized sericin hydrogel

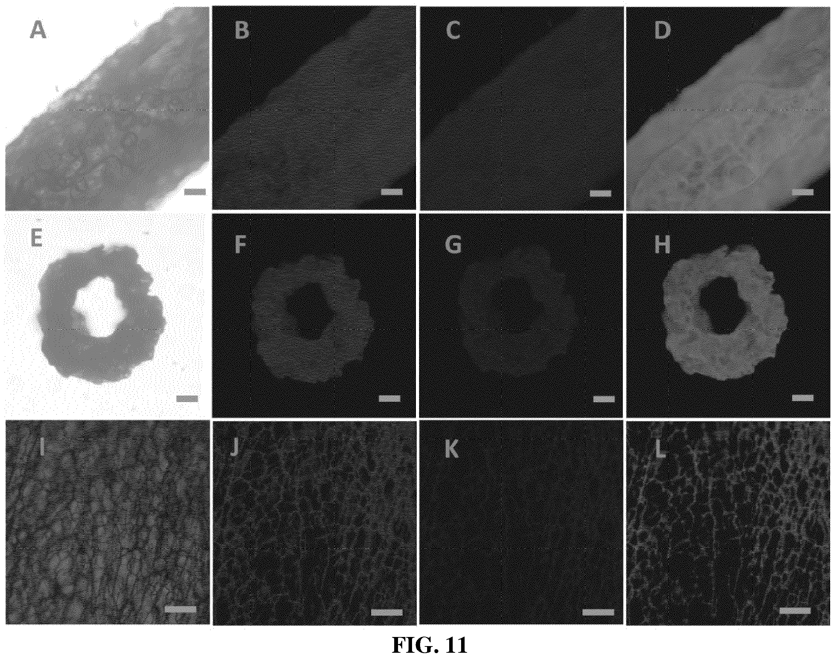

The microstructure of the sericin hydrogel was examined under a fluorescence microscope (Olympus IX71, Japan) at different wavelengths.
As shown in FIG. **11**, the lyophilized scaffold of the sericin hydrogel emit a red fluorescence, a green fluorescence, a blue fluorescence, etc. excited by light sources of different excitation wavelengths.

**Example 6**

1. Use of the lyophilized sericin scaffold as the bioimaging probe

1) The method for preparing the sericin solution was the same as that of Example **1**, and the methods of preparing the hydrogel and the lyophilized scaffold were that same as those in Examples **1**, **4**, and **5**.

2) The prepared sericin hydrogel and the lyophilized scaffold emit different fluorescent lights under different excitation wavelengths, for example, the red fluorescent light appeared under the excitation wavelength of 510-550 nm, the blue fluorescent light appeared under the excitation wavelength of 330-385 nm, and the green fluorescent light appeared under the excitation wavelength of 420 nm.

3) The sericin hydrogel and the lyophilized scaffold were injected into animals and could be tracked by the small animal imaging system in real time (by detecting the fluorescent signals). The sericin hydrogel and the lyophilized scaffold could be used as the fluorescent probe.

2. Experimental analysis

1) Detection of intrinsic fluorescence of sericin conduit and lyophilized sericin hydrogel

The microstructure of the sericin hydrogel was examined under a fluorescence microscope (Olympus IX71, Japan) at different wavelengths.
As shown in FIG. **11**, the lyophilized scaffold of the sericin hydrogel emit a red fluorescence, a green fluorescence, a blue fluorescence, etc. excited by light sources of different excitation wavelengths.

2) Trace analysis of the sericin hydrogel injected *in vivo* by the small animal imaging system

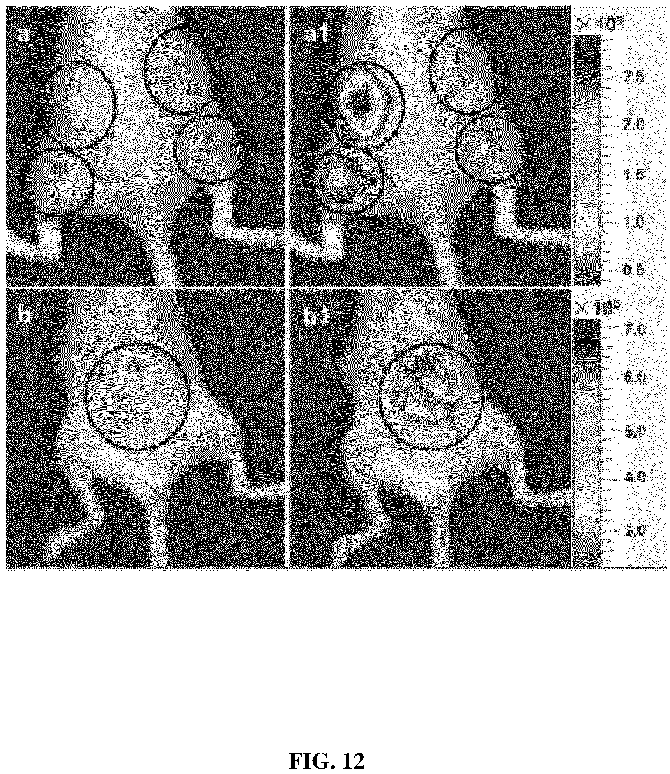

The sericin hydrogels injected into subcutaneous region, intramuscular region, and intraperitoneal region were examined by the small animal imaging system (Xenogen IVIS LuminaII, Caliper Life Sciences, USA).
As shown in FIG. **12**, the sericin hydrogel possessed excellent intrinsic fluorescence, and the hydrogel could be traced *in vivo*.

Example 7

1. Sustained release of horseradish peroxidase (HRP) as a model drug from the sericin hydrogel

1) 4 $\mu$L of 2 $\mu$g/$\mu$L HRP solution and 500 $\mu$L of 2 wt. % sericin solution were mixed for yielding a mixture;

2) 11 $\mu$L of 25 wt. % glutaraldehyde was added to the above mixture to prepare a sample. After being kept at the room temperature for 0.5 hr, the sample was then preserved in the 4°C refrigerator for another 24 hrs.

3) 1 mL of the PBS solution (pH 7.4) was added to sample at the temperature of 37°C.

4) At time points of the former half day, 1st, 2nd, 3rd, 4th, 5th, 6th, 7th, 8th, 9th, 10th, 11th, 12th, 13th, 14th, 15th, 18th, and 20th days, a supernatant was collected and another 1 mL of PBS solution (pH 7.4) was then added.

5) The content of the HRP in a supernatant was measured by the enzyme-linked immunosorbent assay, and a cumulative release rate of the drug was calculated by a ratio of the cumulative HRP content at different time points to the total delivered content of the drug.

2. Experimental analysis

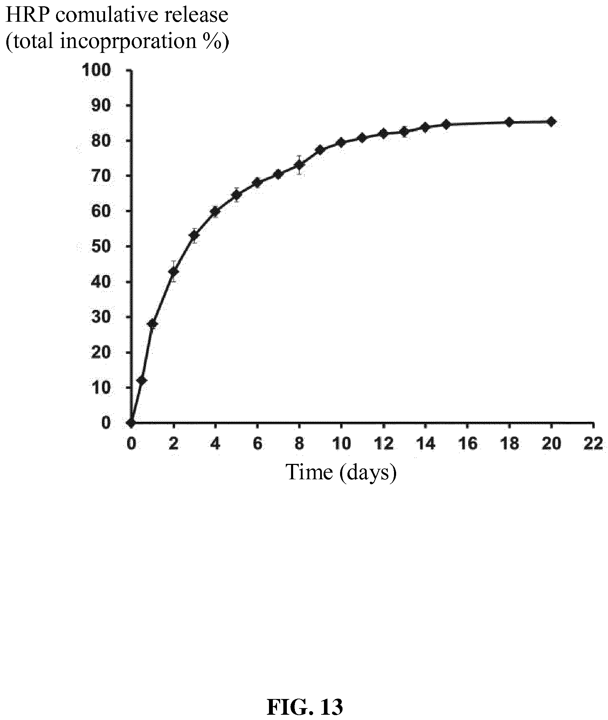

The measuring steps were referred to the above steps 1)-5).
Analysis of the HRP release of the sericin hydrogel was performed.
As shown in FIG. **13**, the release rate of the drug (HRP) was 27.97% within 24 hrs, approximately 50 % in three days, 83.77% in 14 days, and 85.37% in 20 days. The results indicate that the sericin hydrogel possess the sustained drug release property, revealing the possibility for the sericin hydrogel to be used as a drug delivery vehicle *in vivo*.

Example 8

1. Cell culture of human skin epidermal cells (HaCaT) on sericin hydrogel

1) 1.5 mL of 2 wt. % sericin solution and 33 $\mu$L of 25 wt. % glutaraldehyde were added to a cell culture dish, mixed and spread on in the culture dish. After a stable gelation, the cell culture dish spread with the sericin hydrogel was washed by a sterilized PBS solution three times, immersed in 75% ethanol for 1 hr, and washed again by the sterilized PBS solution once. The treated culture dish was kept in the 4°C refrigerator before use.

2) Cells collected from a cell culture flask were suspended and blown and then seeded to the pretreated culture dish, and a culture dish not spread with the sericin hydrogel was utilized as a control group. The cells were cultured in the 1640 culture media in a cell incubator (37°C, 5% $CO_2$, 100% humidity).

3) The cells were photographed under a common light by the microscope Olympus IX71 on the Days 0, 1, and 3.

## 2. Experimental analysis

Comparative analyses of the HaCaT cells cultured in the sericin hydrogel group and the control group were performed.

The cells were cultured by the 1640 culture media, and the detection steps were referred to the above steps 1)-3).

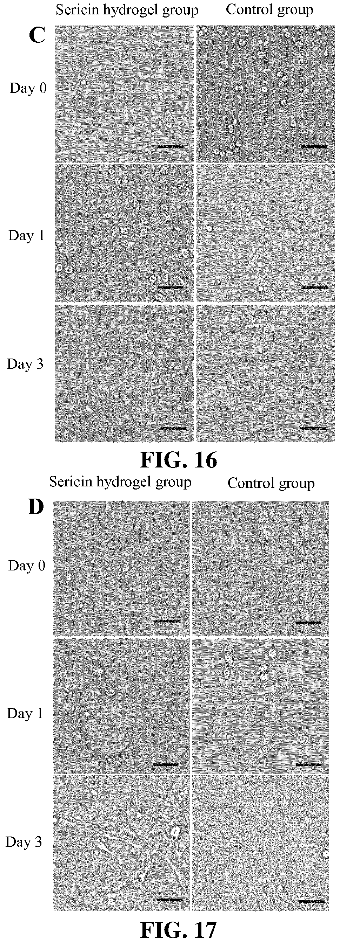

As shown in FIG. **16**, adhesion and proliferation conditions of the HaCaT cells seeded in the control group (at Days 0, 1, and 3) and in the sericin group (at Days 0, 1, and 3) were examined by SEM, and experiment results indicated that the sericin hydrogel is able to well support the adhesion, survival, and proliferation of the HaCaT cells, further proving that the sericin hydrogel is applicable to the skin tissue repair. Scale bar = 50 μm.

## Example 9

1. Cell culture of mouse myoblasts (C2C12) on sericin hydrogel

1) 1.5 mL of 2 wt. % sericin solution and 33 μL of 25 wt. % glutaraldehyde were added to a cell culture dish, mixed and spread on in the culture dish. After a stable gelation, the cell culture dish spread with the sericin hydrogel was washed by a sterilized PBS solution three times, immersed in 75% ethanol for 1 hr, and washed again by the sterilized PBS solution once. The treated culture dish was kept in the 4°C refrigerator before use.

2) Cells collected from a cell culture flask were suspended and blown and then seeded to the pretreated culture dish, and a culture dish not spread with the sericin hydrogel was utilized as a control group. The cells were cultured in the 1640 culture media in a cell incubator (37°C, 5% $CO_2$, 100% humidity).

3) The cells were photographed under a common light by the microscope Olympus IX71 on the Days 0, 1, and 3.

## 2. Experimental analysis

Comparative analyses of the C2C12 cells cultured in the sericin hydrogel group and the control group were performed.

The detection steps were referred to the above steps 1)-3).

As shown in FIG. **17**, adhesion and proliferation conditions of the C2C12 cells seeded in the control group (at Days 0, 1, and 3) and in the sericin group (at Days 0, 1, and 3) were examined by SEM, and experiment results indicated that the sericin hydrogel is able to well support the adhesion, survival, and proliferation of the C2C12 cells, further proving that the sericin hydrogel is applicable to the muscle tissue repair. Scale bar = 50 μm.

## Example 10

1. Cell culture of human embryo kidney cells (HEK293) on sericin hydrogel

1) 1.5 mL of 2 wt. % sericin solution and 33 μL of 25 wt. % glutaraldehyde were added to a cell culture dish, mixed and spread on in the culture dish. After a stable gelation, the cell culture dish spread with the sericin hydrogel was washed by a sterilized PBS solution three times, immersed in 75% ethanol for 1 hr, and washed again by the sterilized PBS solution once. The treated culture dish was kept in the 4°C refrigerator before use.

2) Cells collected from a cell culture flask were suspended and blown and then seeded to the pretreated culture dish, and a culture dish not spread with the sericin hydrogel was utilized as a control group. The cells were cultured in the 1640 culture media in a cell incubator (37°C, 5% $CO_2$, 100% humidity).

3) The cells were photographed under a common light by the microscope Olympus IX71 on the Days 0, 1, and 3.

## 2. Experimental analysis

Comparative analyses of the HEK293 cells cultured in the sericin hydrogel group and the control group were performed.

The detection steps were referred to the above steps 1)-3).

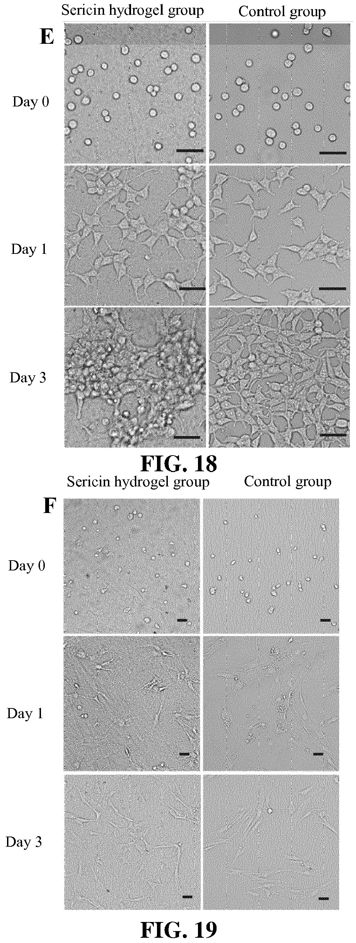

As shown in FIG. **18**, adhesion and proliferation conditions of the HEK293 cells seeded in the control group (at Days 0, 1, and 3) and in the sericin group (at Days 0, 1, and 3) were examined by SEM, and experiment results indicated that the sericin hydrogel is able to well support the adhesion, survival, and proliferation of the HEK293 cells. Moreover, HEK293 cells are excellent tool cells, which further demonstrates that the sericin hydrogel is able to combine the tool cells to pack corresponding treating factors for the tissue repair.

## Example 11

1. Cell culture of human primary embryo skin fibroblasts (CCC-ESF-1) on sericin hydrogel

1) 1.5 mL of 2 wt. % sericin solution and 33 $\mu$L of 25 wt. % glutaraldehyde were added to a cell culture dish, mixed and spread on in the culture dish. After a stable gelation, the cell culture dish spread with the sericin hydrogel was washed by a sterilized PBS solution three times, immersed in 75% ethanol for 1 hr, and washed again by the sterilized PBS solution once. The treated culture dish was kept in the 4°C refrigerator before use.

2) Cells collected from a cell culture flask were suspended and blown and then seeded to the pretreated culture dish, and a culture dish not spread with the sericin hydrogel was utilized as a control group. The cells were cultured in the 1640 culture media in a cell incubator (37°C, 5% $CO_2$, 100% humidity).

3) The cells were photographed under a common light by the microscope Olympus IX71 on the Days 0, 1, and 3.

2. Experimental analysis

Comparative analyses of the CCC-ESF-1 cells cultured in the sericin hydrogel group and the control group were performed.
The cells were cultured by the 1640 culture media, and the detection steps were referred to the above steps 1)-3).
As shown in FIG. **19**, adhesion and proliferation conditions of the CCC-ESF-1 cells seeded in the control group (at Days 0, 1, and 3) and in the sericin group (at Days 0, 1, and 3) were examined by SEM, and experiment results indicated that the sericin hydrogel is able to well support the adhesion, survival, and proliferation of the CCC-ESF-1 cells, further proving that the sericin hydrogel is applicable to the skin tissue repair. Scale bar = 50 $\mu$m.

**Example 12**

1. Cell culture of mouse microglia cells (BV2) on sericin hydrogel

1) 1.5 mL of 2 wt. % sericin solution and 33 $\mu$L of 25 wt. % glutaraldehyde were added to a cell culture dish, mixed and spread on in the culture dish. After a stable gelation, the cell culture dish spread with the sericin hydrogel was washed by a sterilized PBS solution three times, immersed in 75% ethanol for 1 hr, and washed again by the sterilized PBS solution once. The treated culture dish was kept in the 4°C refrigerator before use.

2) Cells collected from a cell culture flask were suspended and blown and then seeded to the pretreated culture dish, and a culture dish not spread with the sericin hydrogel was utilized as a control group. The cells were cultured in the DMEM/F12 culture media in a cell incubator (37°C, 5% $CO_2$, 100% humidity).

3) The cells were photographed under a common light by the microscope Olympus IX71 on the Days 0, 1, and 3.

2. Experimental analysis

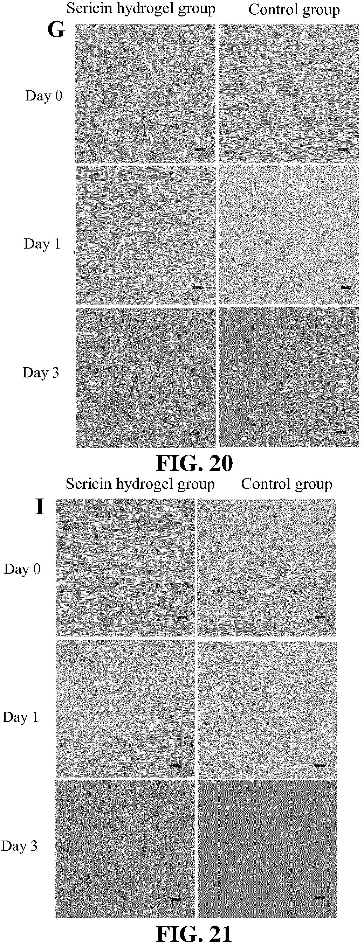

Comparative analyses of the BV2 cells cultured in the sericin hydrogel group and the control group were performed.
The cells were cultured by the DMEM/F12 culture media, and the detection steps were referred to the above steps 1)-3).
As shown in FIG. **20**, adhesion and proliferation conditions of the BV2 cells seeded in the control group (at Days 0, 1, and 3) and in the sericin group (at Days 0, 1, and 3) were examined by SEM, and experiment results indicated that the sericin hydrogel is able to well support the adhesion, survival, and proliferation of the BV2 cells, further proving that the sericin hydrogel is applicable to the nerve tissue repair. Scale bar = 50 $\mu$m.

**Example 13**

1. Cell culture of mouse islet endothelial cells (MS 1) on sericin hydrogel

1) 1.5 mL of 2 wt. % sericin solution and 33 $\mu$L of 25 wt. % glutaraldehyde were added to a cell culture dish, mixed and spread on in the culture dish. After a stable gelation, the cell culture dish spread with the sericin hydrogel was washed by a sterilized PBS solution three times, immersed in 75% ethanol for 1 hr, and washed again by the sterilized PBS solution once. The treated culture dish was kept in the 4°C refrigerator before use.

2) Cells collected from a cell culture flask were suspended and blown and then seeded to the pretreated culture dish, and a culture dish not spread with the sericin hydrogel was utilized as a control group. The cells were cultured in the low glucose DMEM culture media in a cell incubator (37°C, 5% $CO_2$, 100% humidity).

3) The cells were photographed under a common light by the microscope Olympus IX71 on the Days 0, 1, and 3.

2. Experimental analysis

Comparative analyses of the MS1 cells cultured in the sericin hydrogel group and the control group were performed.

The cells were cultured by the low glucose DMEM culture media, and the detection steps were referred to the above steps 1)-3).

As shown in FIG. **21**, adhesion and proliferation conditions of the MS1 cells seeded in the control group (at Days 0, 1, and 3) and in the sericin group (at Days 0, 1, and 3) were examined by SEM, and experiment results indicated that the sericin hydrogel is able to well support the adhesion, survival, and proliferation of the MS1 cells, further proving that the sericin hydrogel is applicable to the blood vessel repair. Scale bar = 50 $\mu$m.

**Example 14**

1. Cell culture of rat Schwann cells (RSC926) on sericin hydrogel

1) 1.5 mL of 2 wt. % sericin solution and 600 $\mu$L of 2 wt. % geniposide were added to a cell culture dish, mixed and spread on in the culture dish. After a stable gelation, the cell culture dish spread with the sericin hydrogel was immersed in 75% ethanol for 1 hr, separated from ethanol, and washed by the sterilized PBS solution once. The treated culture dish was kept in the 4°C refrigerator before use.

2) Cells collected from a cell culture flask were suspended and blown and then seeded to the pretreated culture dish, and a culture dish not spread with the sericin hydrogel was utilized as a control group. The cells were cultured in the high glucose DMEM culture media in a cell incubator (37°C, 5% $CO_2$, 100% humidity).

3) The cells were photographed under a common light by the microscope Olympus IX71 on the Day 1 and Day 2.

2. Experimental analysis

Comparative analyses of the RSC926 cells cultured in the sericin hydrogel group and the control group were performed.

The cells were cultured by the low glucose DMEM culture media, and the detection steps were referred to the above steps 1)-3).

As shown in FIG. **17**, adhesion and proliferation conditions of the RSC926 cells seeded in the control group (at Day 1 and Day 2) and in the sericin group (at Day 1 and Day 2) were examined by SEM, and experiment results indicated that the sericin hydrogel is able to well support the adhesion, survival, and proliferation of the RSC926 cells, further proving that the sericin hydrogel is applicable to the peripheral nerve injury repair.

Scale bar = 100 $\mu$m.

**Example 15**

1. Cell culture of rat cardiac myocytes (H9C2) on sericin hydrogel

1) 1.5 mL of 2 wt. % sericin solution and 600 $\mu$L of 2 wt. % geniposide were added to a cell culture dish, mixed and spread on in the culture dish. After a stable gelation, the cell culture dish spread with the sericin hydrogel was immersed in 75% ethanol for 1 hr, separated from ethanol, and washed by the sterilized PBS solution once. The treated culture dish was kept in the 4°C refrigerator before use.

2) Cells collected from a cell culture flask were suspended and blown and then seeded to the pretreated culture dish, and a culture dish not spread with the sericin hydrogel was utilized as a control group. The cells were cultured in the high glucose DMEM culture media in a cell incubator (37°C, 5% $CO_2$, 100% humidity).

3) The cells were photographed under a common light by the microscope Olympus IX71 on the 1st days.

2. Experimental analysis

Comparative analyses of the H9C2 cells cultured in the sericin hydrogel group and the control group were performed.

The cells were cultured by the low glucose DMEM culture media, and the detection steps were referred to the above steps 1)-3).

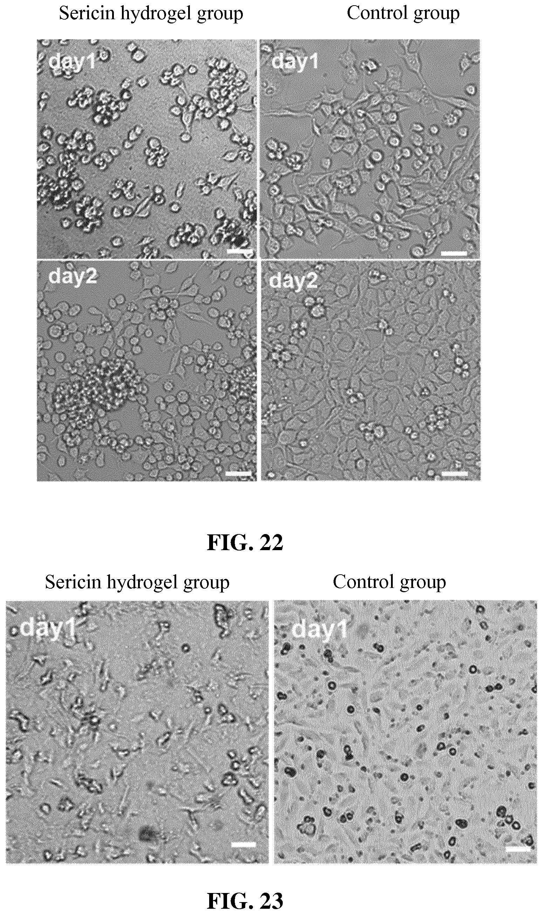

As shown in FIG. **23,** adhesion and proliferation conditions of the H9C2 cells seeded in the control group (1st day) and in the sericin group (1st day) were examined by SEM, and experiment results indicated that the sericin hydrogel is able to well support the adhesion, survival, and proliferation of the H9C2 cells, further proving that the sericin hydrogel is applicable to the cardiac tissue repair. Scale bar = 100 $\mu$m.

**Example 16**

1. Cell culture of HUVECs on sericin hydrogel (ECV304 cells adopted for cell adhesion experiment and EA.hy926 cells adopted for cell viability)

1) 1.5 mL of 2 wt. % sericin solution and 33 $\mu$L of 25 wt. % glutaraldehyde were added to a cell culture dish, mixed and spread on in the culture dish. After a stable gelation, the cell culture dish spread with the sericin hydrogel was washed by a sterilized PBS solution three times, immersed in 75% ethanol for 1 hr, and washed again by the sterilized PBS solution once. The treated culture dish was kept in the 4°C refrigerator before use.

2) Cells collected from a cell culture flask were suspended and blown and then seeded to the pretreated culture dish, and a culture dish not spread with the sericin hydrogel was utilized as a control group. The cells were cultured in the high glucose DMEM culture media in a cell incubator (37°C, 5% $CO_2$, 100% humidity).

3) Non-adherent cells were gently washed down by the PBS solution (pH 7.4) and counted by a cell count plate at the two time points of 4 hrs and 8 hrs after the seeding. The cell adhesion rate was calculated by a ratio of a difference between the originally seeded cell number and the non-adherent cell number to the originally seeded cell number.

4) Cell viabilities were measured by the MTT method after 2.5 days and 4.5 days of the cell culture. The cells were photographed at Days 0, 1, and 3 under the common light by the microscope Olympus IX71.

5) The cells were stained by Rhodamine phalloidin and 4',6-diamidino-2-phenylindole respectively after two days of cell culture, and was observed and photographed by confocal laser scanning microscopy (CLSM) (iNikon AlSi, Japan).

2. Experimental analysis

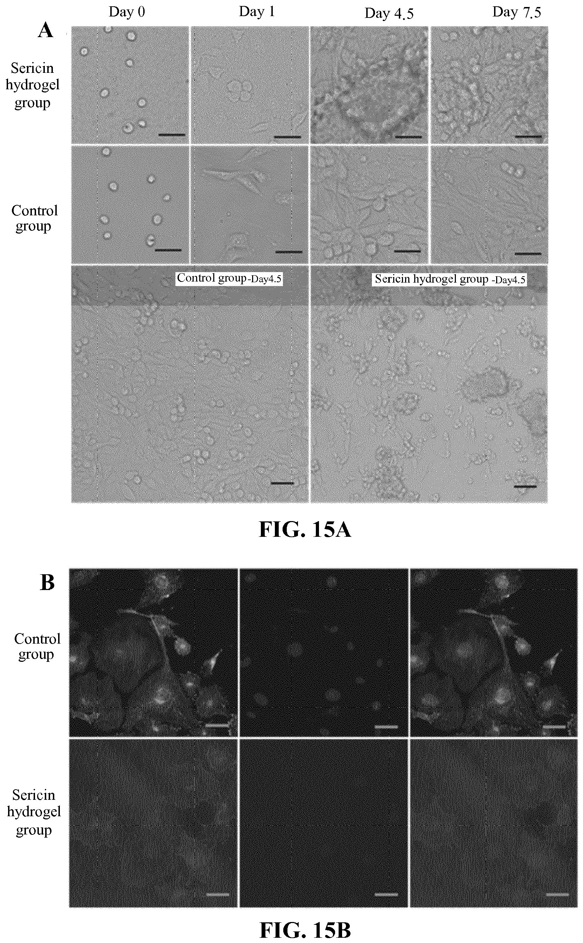

FIGs. **15A-15B** illustrate the adhesion and proliferation of the HUVECs observed by the ordinary electron microscope and the CLSM. FIG. **15A** shows the ordinary electron micrograms of the HUVECs in the control group (at Day 0, 1, 4.5, and 7.5 after seeding) and on the sericin hydrogel group (at Day 0, 1, 4.5, and 7.5 after seeding); and FIG. **15B** shows the CLSM pictures of the HUVECs in the control group and on the sericin hydrogel group at Day 1 after the seeding. The experiment results demonstrates that the sericin hydrogel is able to well support the adhesion, survival, and proliferation of the HUVECs, further proving that the sericin hydrogel is applicable to the blood vessel repair. Scale bar = 50 μm.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

**Claims**

1. A method for preparing a sericin hydrogel, the method comprising:

   1) weighing a cocoon of a fibroin-deficient mutant silkworm, *Bombyx mori*, extracting the cocoon by an aqueous solution of LiBr or LiCl, dialyzing an extracted solution whereby yielding a sericin solution having a concentration of a non-degraded sericin of between 0.1 and 4 wt. %; and
   2) concentrating the sericin solution to the concentration of between 1.5 and 10 wt. %, adding a crosslinking agent to the concentrated sericin solution in a ratio of between 2 and 500 μL of the crosslinking agent per each milliliter of the sericin solution added, fully blending the crosslinking agent with the concentrated sericin solution, and keeping a resulting mixture at a temperature of between 4 and 45°C for between 5 s and 36 hrs whereby yielding a hydrogel.

2. The method of claim 1, wherein the crosslinking agent is selected from the group consisting of glutaraldehyde, malondialdehyde, and geniposide.

3. The method of claim 1 or 2, wherein a concentration of the crosslinking agent is between 1 and 25 wt. %.

4. The method of claim 1 or 2, wherein in 1), the sericin solution is prepared as follows:

   a) weighing the cocoon of the fibroin-deficient mutant silkworm, *Bombyx mori*, cutting the cocoon into pieces, washing and dehydrating the cocoon pieces;
   b) immersing the cocoon pieces obtained from step a) into the aqueous solution of LiBr or LiCl at the temperature of between 25 and 50°C for dissolving the sericin, wherein each gram of the cocoon pieces corresponds to between 20 and 100 mL of the aqueous solution of LiBr or LiCl having a concentration of between 6 and 8 mol/L;
   c) centrifuging a mixture of step b), removing insoluble substances therefrom whereby yielding a clarified solution;
   d) adding a Tris-HCl buffer solution having a concentration of 1 mol/L and a pH value of between 8.0 and 11.0 to the clarified solution obtained in c) with a volume ratio of the Tris-HCl buffer solution to the clarified solution of 1:4, dialyzing a mixed solution whereby yielding the sericin solution; and
   e) removing a precipitate from the sericin solution by centrifuging, and concentrating the sericin solution to the concentration of between 1.5 and 10 wt. %.

5. The method of claim 1 or 2, comprising:

a) weighing the cocoon of the fibroin-deficient mutant silkworm, cutting the cocoon into pieces in a size of 1 cm$^2$, washing the cocoon pieces three times, and dehydrating the cocoon pieces;
b) immersing the cocoon pieces obtained from step a) into the aqueous solution of LiBr at a temperature of 35°C for 24 hrs for dissolving the sericin, wherein each gram of the cocoon pieces corresponds to 40 mL of the aqueous solution of LiBr having a concentration of 6 mol/L;
c) centrifuging a mixture of step b), removing insoluble substances therefrom whereby yielding a clarified solution;
d) adding a Tris-HCl buffer solution having a concentration of 1 mol/L and a pH value of 9.0 to the clarified solution obtained in c) with a volume ratio of the Tris-HCl buffer solution to the clarified solution of 1:4, dialyzing a mixed solution whereby yielding the sericin solution;
e) removing a precipitate from the sericin solution obtained in d) by centrifuging, and concentrating the sericin solution to the concentration of between 1.5 and 10 wt. %; and
f) adding glutaraldehyde to the sericin solution, blending glutaraldehyde with the sericin solution, and keeping a resulting mixture at 37°C for 5 min whereby yielding a hydrogel, wherein each milliliter of the sericin solution corresponds to between 2 and 100 μL of between 20 and 25 wt. % of glutaraldehyde.

6. A method for preparing a biomedical material comprising admixing a sericin hydrogel prepared by the method of any one of claims 1-5.

7. The method of claim 6, wherein the sericin hydrogel is used in the following aspects:

1) damage repair and disease treatment including, but not limited to, skin injury, muscle injury, vascular injury, nerve injury, and myocardial injury;
2) tissue repair by combining the sericin hydrogel with tool cells to pack corresponding treating factors; and
3) as a growth factor, a drug, a cell carrier, or a scaffold.

8. A method for preparing a lyophilized sericin scaffold, the method comprising:

1) freezing the sericin hydrogel at a temperature beneath zero; and
2) vacuum drying the frozen sericin hydrogel whereby yielding a lyophilized sericin scaffold.

9. A method for preparing a biomedical material comprising admixing a lyophilized sericin scaffold prepared by the method of claim 8.

10. A method for preparing a fluorescent probe material comprising admixing a sericin hydrogel prepared by the method of any one of claims 1-5.

11. A method for preparing a biomedical material comprising admixing a sericin hydrogel prepared by the method of any one of claims 1-5.

**FIG. 1**

Gelation time (seconds)

Concentration of sericin hydrogels

**FIG. 2**

Degaree of crosslinking (%)

Time (hrs)

**FIG. 3**

Compressive strength (kPa)

Types of hydrogels

**FIG. 4A**

Compressive strength (kPa)

Types of hydrogels

**FIG. 4B**

Degrading of sericin hydrogels (%)

**FIG. 5**

**FIG. 6**

Swellling (%)

**FIG. 7**

% Equilibrium water content

**FIG. 8**

DSC (mW/mg)

TEMP (°C)

**FIG. 9**

Wave number (cm⁻¹)

**FIG. 10**

**FIG. 11**

**FIG. 12**

HRP comulative release
(total incoprporation %)

Time (days)

**FIG. 13**

Adhesion (%)

FIG. 14A

Cell viability
O.D. at 570nm

FIG. 14B

**FIG. 15A**

**FIG. 15B**

Sericin hydrogel group    Control group

**C**

Day 0

Day 1

Day 3

**FIG. 16**

Sericin hydrogel group    Control group

**D**

Day 0

Day 1

Day 3

**FIG. 17**

FIG. 18

FIG. 19

FIG. 20

FIG. 21

Sericin hydrogel group          Control group

**FIG. 22**

Sericin hydrogel group          Control group

**FIG. 23**

## INTERNATIONAL SEARCH REPORT

| International application No. |
|---|
| PCT/CN2014/075298 |

**A. CLASSIFICATION OF SUBJECT MATTER**

C08H 1/00 (2006.01) i; C08J 3/24 (2006.01) i; A61L 27/22 (2006.01) i

According to International Patent Classification (IPC) or to both national classification and IPC

**B. FIELDS SEARCHED**

Minimum documentation searched (classification system followed by classification symbols)

C08H; C08J; A61L

Documentation searched other than minimum documentation to the extent that such documents are included in the fields searched

Electronic data base consulted during the international search (name of data base and, where practicable, search terms used)

WPI, EPODOC, CNPAT, CNKI, ISI Web of knowledge: sericin, hydrogel, gel, aquogel, dialysis, LiBr, LiCl, concentration, cross-link,

glutaraldehyde, glutaric dialdehyde, support, bracket, cryodesiccation, freeze-drying, lyophilization, fluorescence, probe, medical

**C. DOCUMENTS CONSIDERED TO BE RELEVANT**

| Category* | Citation of document, with indication, where appropriate, of the relevant passages | Relevant to claim No. |
|---|---|---|
| PX | CN 103951831 A (UNION HOSPITAL TONGJI MEDICAL COLLAGE HUAZHONG UNIVERSITY OF SCIENCE AND TECHNOLOGY) 30 July 2014 (30.07.2014) | 1-11 |
| Y | ZHAO, Xiaojing and ZHANG, Keqin, 'RecoveryTechnologies of Sericin from Silk Degumming Wastewater', Dyeing & Finishing, 31 May 2013 (31.05.2013), page 50, section 1.3.1, and page51, section 1.4.4 | 1-9, 11 |
| Y | LI, Haibin, 'Preparation and Cytocompatibility Study of Poly (ε-Caprolactone)/Silk Sericin Nanofibrous Scaffolds', China Master's Theses Full-test Database (electronic journal), no. 03, 15 April 2011 (15.04.2011), page 18, section 3.3.2 | 1-9, 11 |
| Y | ZHANG, Haiping, 'Preparation and Characterization of Spongy Hydrogels from Aqueous Bombyx Mori Sericin', China Doctor's Theses Full-test Database (electronic journal), no. 04, 15 May 2011 (15.05.2011), page 1, section 1.1, page 7, section 1.2.1.1, page 13, section 1.3.1 | 6-9, 11 |

☐ Further documents are listed in the continuation of Box C.     ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 25 November 2014 | 03 December 2014 |

| Name and mailing address of the ISA State Intellectual Property Office of the P. R. China No. 6, Xitucheng Road, Jimenqiao Haidian District, Beijing 100088, China Facsimile No. (86-10) 62019451 | Authorized officer LI, Yang Telephone No. (86-10) 62411031 |
|---|---|

Form PCT/ISA /210 (second sheet) (July 2009)

**INTERNATIONAL SEARCH REPORT**
Information on patent family members

| | International application No. |
|---|---|
| | PCT/CN2014/075298 |

| Patent Documents referred in the Report | Publication Date | Patent Family | Publication Date |
|---|---|---|---|
| CN 103951831 A | 30 July 2014 | None | |

Form PCT/ISA /210 (patent family annex) (July 2009)